# EUROPEAN PATENT SPECIFICATION

(11) **EP 1 307 562 B1**
(45) Date of publication and mention of the grant of the patent: **15.12.2004**
(21) Application number: 01954016.0
(22) Date of filing: 06.07.2001
(51) Int. Cl.: C12N 15/31, C12P 13/08, C12N 1/21, C07K 14/34

(54) **NUCLEOTIDE SEQUENCES WHICH CODE FOR THE LYSR3 GENE**
NUKLEOTID SEQUENZEN KODIEREND FÜR DAS LYSR3 GEN
SEQUENCES DE NUCLEOTIDES CODANT POUR LE GENE LYSR3

(30) Priority: 10.08.2000 DE 10039049; 31.05.2001 US 867537
(43) Date of publication of application: 07.05.2003
(73) Proprietor: Degussa AG, 40474 Düsseldorf (DE)
(72) Inventor: MÖCKEL, Bettina, 40597 Düsseldorf (DE); KREUTZER, Caroline, 49326 Melle (DE)
(86) International application number: PCT/EP2001/007765
(87) International publication number: WO 2002/012505

(56) References cited:
- EP-A- 1 108 790
- WO-A-01/00842
- WO-A-99/18228
- VRLJIC M ET AL: "A NEW TYPE OF TRANSPORT WITH A NEW TYPE OF CELLULAR FUNCTION: L-IYSINE EXPORT FROM CORYNEBACTERIUM GLUTAMICUM" MOLECULAR MICROBIOLOGY, BLACKWELL SCIENTIFIC, OXFORD, GB, vol. 22, no. 5, 1 December 1996 (1996-12-01), pages 815-826, XP000675494 ISSN: 0950-382X
- EIKMANNS ET AL.: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK, vol. 46, 1993, pages 145-163, XP000918559

## Description

The invention provides isolated coryneform bacteria, wherein the expression is eliminated of nucleotide sequences which code for the lysR3 polypeptide and a process for the fermentative preparation of L-lysine and L-valine. The lysR3 gene codes for the LysR3 protein, which is a transcription regulator of the LysR family.

### Prior Art

L-Amino acids, in particular L-lysine and L-valine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and which produce amino acids are obtained in this manner.

Methods of the recombinant DNA technique have also been employed for some years for improving the strain of Corynebacterium strains which produce L-amino acids.

M. Vrljic et al. (Molecular Microbiology, Blackwell Scientific, Oxford, GB, Vol. 22, No. 5, 1. December 1996 (1996-12-01), pages 815 - 826, XP 000675794 ISSN: 0950-382X describe a corynebacterial strain lacking lysG, a member of the LysR family of regulators. However, there is no significant sequence homology between LysG and LysR and the effect on lysine production shown there was related to disruption of the export protein LysE rather than related to disruption of LysG.

### Object of the Invention

The inventors had the object of providing new measures for improved fermentative preparation of L-lysine and L-valine.

### Description of the Invention

The invention provides:
an isolated coryneform bacterium, in which the expression of a polynucleotide encoding a polypeptide having an amino acid sequence which is at least 90 % identical to the amino acid sequence set out in SEQ ID NO:2 is eliminated, wherein the polypeptide has the function of a transcription regulator of the lysR family.

Preferably, said polypeptide has the amino acid sequence of SEQ ID NO:2.

The polypeptide is preferably encoded by the nucleotide sequence shown in SEQ ID No.1.

The invention also provides:
a vector pCR2.11ysR3int, deposited in Escherichia coli DSM 13618 at the DSMZ [German Collection of Microorganisms and Cell Cultures], Braunschweig (Germany);
and coryneform bacteria which contain an insertion or deletion in the lysR3 gene, in particular using the vector pCR2.11ysR3int.

Described are polynucleotides which substantially comprise a polynucleotide sequence, which are obtainable by screening by means of hybridization of a corresponding gene library, which comprises the complete gene with the polynucleotide sequence corresponding to SEQ ID No. 1, with a probe which comprises the sequence of the polynucleotide mentioned, according to SEQ ID No. 1 or a fragment thereof, and isolation of the DNA sequence mentioned.

Polynucleotide sequences according to the invention are suitable as hybridization probes for RNA, cDNA and DNA, in order to isolate, in the full length, nucleic acids or polynucleotides or genes which code for the LysR3 protein or to isolate those nucleic acids or polynucleotides or genes which have a high similarity with the sequence of the lysR3 gene.

Polynucleotide sequences according to the description are furthermore suitable as primers with the aid of which DNA of genes which code for the LysR3 protein can be prepared with the polymerase chain reaction (PCR).

Such oligonucleotides which serve as probes or primers comprise at least 30, preferably at least 20, very particularly preferably at least 15 successive nucleotides. Oligonucleotides which have a length of at least 40 or 50 nucleotides are also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

"Polypeptides" are understood as meaning peptides or proteins which comprise two or more amino acids bonded via peptide bonds.

The described polypeptides include a polypeptide according to SEQ ID No. 2, in particular those with the biological activity of the LysR3 protein, and also those which are at least 90% to 95% identical to the polypeptide according to SEQ ID No. 2 and have the activity mentioned.

The invention moreover provides a process for the fermentative preparation of L-lysine and L-valine using coryneform bacteria which in particular already produce said amino acids, and in which the nucleotide sequences which code for the lysR3 gene are eliminated.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

The microorganisms which the present invention provides can prepare L-lysine and L-valine, from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
or L-amino acid-producing mutants or strains prepared therefrom, such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DM58-1
Corynebacterium glutamicum DG52-5
Corynebacterium glutamicum DSM 5714 and
Corynebacterium glutamicum DSM 12866
or such as, for example, the L-valine-producing strains
Corynebacterium glutamicum DSM 12455
Corynebacterium glutamicum FERM-P 9325
Brevibacterium lactofermentum FERM-P 9324
Brevibacterium lactofermentum FERM-BP 1763.

The lysR3 gene of C. glutamicum codes for the LysR3 protein, which is a transcription regulator of the LysR family.

To isolate the lysR3 gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie [Genes and Clones, An Introduction to Genetic Engineering] (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575). Börmann et al. (Molecular Microbiology 6(3), 317-326)) (1992)) in turn describe a gene library of C. glutamicum ATCC13032 using the cosmid pHC79 (Hohn and Collins, 1980, Gene 11, 291-298).

To prepare a gene library of C. glutamicum in E. coli it is also possible to use plasmids such as pBR322 (Bolivar, 1979, Life Sciences, 25, 807-818) or pUC9 (Vieira et al., 1982, Gene, 19:259-268). Suitable host are, in particular, those E. coli strains which are restriction- and recombination-defective, such as, for example, the strain DH5α (Jeffrey H. Miller: "A Short Course in Bacterial Genetics, A Laboratory Manual and Handbook for Escherichia coli and Related Bacteria", Cold Spring Harbor Laboratory Press, 1992).

The long DNA fragments cloned with the aid of cosmids or other λ-vectors can then be subcloned in turn into the usual vectors suitable for DNA sequencing.

Methods of DNA sequencing are described, inter alia, by Sanger et al. (Proceedings of the National Academy of Sciences of the United States of America USA, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The DNA sequence of C. glutamicum which codes for the lysR3 protein and which, as SEQ ID No. 1, is described in the present invention has been found in this manner. The amino acid sequence of the corresponding protein has furthermore been derived from the present DNA sequence by the methods described above. The resulting amino acid sequence of the lysR3 gene product is shown in SEQ ID No. 2.

Coding DNA sequences which result from SEQ ID No. 1 by the degeneracy of the genetic code are also known. In the same way, DNA sequences which hybridize with SEQ ID No. 1 or parts of SEQ ID No. 1 are described by the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are described.

Finally, DNA sequences which are prepared by the polymerase chain reaction (PCR) using primers which result from SEQ ID No. 1 are described. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). Instructions for amplification of DNA sequences with the aid of the polymerase chain reaction (PCR) can be found by the expert, inter alia, in the handbook by Gait: Oligonucleotide synthesis: A Practical Approach (IRL Press, Oxford, UK, 1984) and in Newton and Graham: PCR (Spektrum Akademischer Verlag, Heidelberg, Germany, 1994).

In the work on the present invention, it has been found that coryneform bacteria produce L-lysine and L-valine, in an improved manner after elimination of the lysR3 gene.

To achieve an elimination, either the expression of the lysR3 gene or the catalytic properties of the enzyme protein can be eliminated. The two measures can optionally be combined.

The reduction in gene expression can take place by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in pátek et al. (Microbiology 142: 1297 (1996)), Vasicova et al. (Journal of Bacteriology 181: 6188 (1999)) and in known textbooks of genetics and molecular biology, such as e.g. the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms [Threonine dehydratase from Corynebacterium glutamicum: Cancelling the allosteric regulation and structure of the enzyme]", Reports from the Jülich Research Center, Jül-2906, ISSN09442952, Jülich, Germany, 1994). Summarizing descriptions can be found in known textbooks of genetics and molecular biology, such as e.g. that by Hagemann ("Allgemeine Genetik [General Genetics]", Gustav Fischer Verlag, Stuttgart, 1986).

Possible mutations are transitions, transversions, insertions and deletions. Depending on the effect of the amino acid exchange on the enzyme activity, missense mutations or nonsense mutations are referred to. Insertions or deletions of at least one base pair (bp) in a gene lead to frame shift mutations, as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stuttgart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986).

A common method of mutating genes of C. glutamicum is the method of gene disruption and gene replacement described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 (1992)), pGEM-T (Promega corporation, Madison, WI, USA), pCR2.1-TOPO (Shuman (1994). Journal of Biological Chemistry 269:32678-84; US Patent 5,487,993), pCR®Blunt (Invitrogen, Groningen, Holland; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of C. glutamicum.

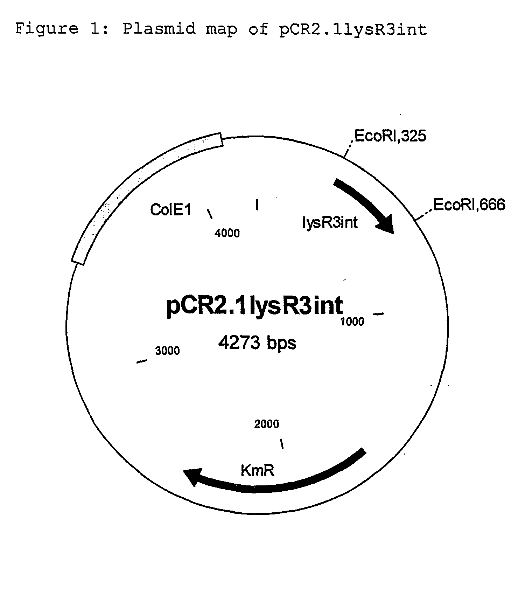

Figure 1 shows by way of example the plasmid vector pCR2.1lysR3int, with the aid of which the lysR3 gene can be disrupted or eliminated.

In the method of gene replacement, a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "crossover" event which effects integration and a suitable second "cross-over" event which effects excision in the target gene or in the target sequence, the incorporation of the mutation or of the allele is achieved. This method was used, for example, by Peters-Wendisch et al.(Microbiology 144, 915 - 927 (1998)) to eliminate the pyc gene of C. glutamicum by a deletion.

A deletion, insertion or a base exchange can be incorporated into the lysR3 gene in this manner.

In addition, it may be advantageous for the production of L-lysine and L-valine to enhance, in particular to over-express, one or more enzymes of the particular biosynthesis pathway, of glycolysis, of anaplerosis, of the pentose phosphate cycle or of amino acid export, in addition to attenuation of the lysR3 gene.

Thus, for example, for the preparation of L-lysine, at the same time one or more of the genes chosen from the group consisting of
- the dapA gene which codes for dihydrodipicolinate synthase (EP-B 0 197 335),
- the eno gene which codes for enolase (DE: 19947791.4),
- the zwf gene which codes for the zwf gene product (JP-A-09224661),
- the pyc gene which codes for pyruvate carboxylase (Peters-Wendisch et al.(Microbiology 144, 915 - 927 (1998))
- the lysE gene which codes for a protein for lysine export (DE-A-195 48 222)
can be enhanced, in particular over-expressed.

It may furthermore be advantageous for the production of L-lysine, in addition to the elimination of the lysR3 gene, at the same time for one or more of the genes chosen from the group consisting of
- the pck gene which codes for phosphoenol pyruvate carboxykinase (DE 199 50 409.1, DSM 13047),
- the pgi gene which codes for glucose 6-phosphate isomerase(US 09/396,478, DSM 12969),
- the poxB gene which codes for pyruvate oxidase (DE:1995 1975.7, DSM 13114)
to be attenuated.

Thus, for example, for the production of L-valine
- at the same time the ilvBN gene which codes for acetohydroxy-acid synthase (Keilhauer et al., (1993) Journal of Bacteriology 175: 5595-5603), or
- at the same time the ilvD gene which codes for dihydroxy-acid dehydratase (Sahm and Eggeling (1999) Applied and Environmental Microbiology 65: 1973-1979), or
- at the same time the mqo gene which codes for malate:quinone oxidoreductase (Molenaar et al., European Journal of Biochemistry 254, 395-403 (1998))
can be over-expressed.

In addition to elimination of the lysR3 gene it may furthermore be advantageous, for the production of L-lysine and L-valine, to eliminate undesirable side reactions, (Nakayama: "Breeding of Amino Acid Producing Microorganisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of L-lysine and L-valine. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as, for example, soya oil, sunflower oil, groundnut oil and coconut fat, fatty acids, such as, for example, palmitic acid, stearic acid and linoleic acid, alcohols, such as, for example, glycerol and ethanol, and organic acids, such as, for example, acetic acid, can be used as the source of carbon. These substance can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as, for example, magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH. Antifoams, such as, for example, fatty acid polyglycol esters, can be employed to control the development of foam. Suitable substances having a selective action, such as, for example, antibiotics, can be added to the medium to maintain the stability of plasmids. To maintain aerobic conditions, oxygen or oxygen-containing gas mixtures, such as, for example, air, are introduced into the culture. The temperature of the culture is usually 20°C to 45°C, and preferably 25°C to 40°C. Culturing is continued until a maximum of the desired product has formed. This target is usually reached within 10 hours to 160 hours.

Methods for the determination of L-amino acids are known from the prior art. The analysis can thus be carried out, for example, as described by Spackman et al. (Analytical Chemistry, 30, (1958), 1190) by anion exchange chromatography with subsequent ninhydrin derivation, or it can be carried out by reversed phase HPLC, for example as described by Lindroth et al. (Analytical Chemistry (1979) 51: 1167-1174).

The following microorganism has been deposited at the Deutsche Sammlung für Mikroarganismen und Zellkulturen (DSMZ = German Collection of Microorganisms and Cell Cultures, Braunschweig, Germany) in accordance with the Budapest Treaty:
- Escherichia coli strain TOP10F/pCR2.1lysR3int as DSM 13618.

The process according to the invention is used for the fermentative preparation of L-lysine and L-valine.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from C. glutamicum ATCC 13032

Chromosomal DNA from C. glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al., 1987, Proceedings of the National Academy of Sciences, USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Res. 16:1563-1575). the cells were taken up in 10 mM MgSO₄ and mixed with an aliquot of the phage suspension. The infection and titering of the cosmid library were carried out as described by Sambrook et al. (1989, Molecular Cloning: A Laboratory Manual, Cold Spring Harbor), the cells being plated out on LB agar (Lennox, 1955, Virology, 1:190) + 100 µg/ml ampicillin. After incubation overnight at 37°C, recombinant individual clones were selected.

### Example 2

### Isolation and sequencing of the lysR3 gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, The Netherlands, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences, U.S.A., 87:4645-4649) and plated out on LB agar (Lennox, 1955, Virology, 1:190) with 50 µg/ml zeocin.

The plasmid preparation of the recombinant clones was carried out with Biorobot 9600 (Product No. 900200, Qiagen, Hilden, Germany). The sequencing was carried out by the dideoxy chain termination method of Sanger et al. (1977, Proceedings of the National Academies of Sciences, U.S.A., 74:5463-5467) with modifications according to Zimmermann et al. (1990, Nucleic Acids Research, 18:1067). The "RR dRhodamin Terminator Cycle Sequencing Kit" from PE Applied Biosystems(Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analyses were prepared with the XNIP program (Staden, 1986, Nucleic Acids Research, 14:217-231). Further analyses were carried out with the "BLAST search program" (Altschul et al., 1997, Nucleic Acids Research, 25:3389-3402) against the non-redundant databank of the "National Center for Biotechnology Information" (NCBI, Bethesda, MD, USA).

The resulting nucleotide sequence is shown in SEQ ID No. 1. Analysis of the nucleotide sequence showed an open reading frame of 633 base pairs, which was called the lysR3 gene. The lysR3 gene codes for a polypeptide of 210 amino acids.

### Example 3

### Preparation of an integration vector for integration mutagenesis of the lysR3 gene

From the strain ATCC 13032, chromosomal DNA was isolated by the method of Eikmanns et al. (Microbiology 140: 1817 - 1828 (1994)). On the basis of the sequence of the lysR3 gene known for C. glutamicum from example 2, the following oligonucleotides were chosen for the polymerase chain reaction:

The primers shown were synthesized by MWG Biotech (Ebersberg, Germany) and the PCR reaction was carried out by the standard PCR method of Innis et al. (PCR protocols. A guide to methods and applications, 1990, Academic Press) with Pwo-Polymerase from Boehringer. With the aid of the polymerase chain reaction, an internal fragment of the lysR3 gene 323 bp in size was isolated, this being shown in SEQ ID No. 3.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991) Bio/Technology 9:657-663).

The E. coli strain TOP10F was then electroporated with the ligation batch (Hanahan, In: DNA cloning. A practical approach. Vol. I, IRL-Press, Oxford, Washington DC, USA, 1985). Selection for plasmid-carrying cells was made by plating out the transformation batch on LB agar (Sambrook et al., Molecular Cloning: A Laboratory Manual. 2^{nd} Ed., Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.11ysR3int.

### Example 4

### Integration mutagenesis of the lysR3 gene in the lysine producer DSM 5715 and in the valine producer FERM BP-1763

The vector pCR2.11ysR3int mentioned in example 3 was electroporated by the electroporation method of Tauch et al.(FEMS Microbiological Letters, 123:343-347 (1994)) into Corynebacterium glutamicum DSM 5715 and Brevibacterium lactofermentum FERM BP-1763. The strain DSM 5715 is an AEC-resistant lysine producer. The strain FERM BP-1763 is a valine producer in need of isoleucine and methionine. The vector pCR2.11ysR3int cannot replicate independently in DSM 5715 or FERM BP-1763 and is retained in the cell only if it has integrated into the chromosome of DSM 5715 or FERM BP-1763. Selection of clones with pCR2. 1lysR3int integrated into the chromosome was carried out by plating out the electroporation batch on LB agar (Sambrook et al., Molecular Cloning: A Laboratory Manual. 2^{nd} Ed., Cold Spring Harbor Laboratory Press, Cold'Spring Harbor, N.Y.), which had been supplemented with 15 mg/l kanamycin.

For detection of the integration, the lysR3int fragment was labeled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes SalI, SacI and HindIII. The fragments formed were separated by agarose gel electrophoresis and hybridized at 68°C with the Dig hybrization kit from Boehringer. The plasmid pCR2. 1lysR3int mentioned in example 3 had been inserted into the chromosome of DSM5715 and FERM BP-1763 within the chromosomal lysR3 gene. The strains-were called DSM5715::pCR2.11ysR3int and FERM BP-1763::pCR2.1lysR3int.

### Example 5

### Preparation of L-lysine and L-valine

The C. glutamicum and B. lactofermentum strains DSM5715::pCR2.1lysR3int and FERM BP-1763::pCR2.1lysR3int obtained in example 4 were cultured in a nutrient medium suitable for the production of L-lysine and L-valine and the L-lysine and L-valine content in the culture supernatant was determined.

For this, the strains were first incubated on an agar plate with the corresponding antibiotic (brain-heart agar with kanamycin (25 mg/l) for 24 hours at 33°C. Starting from this agar plate culture, a pre-culture was seeded (10 ml medium in a 100 ml conical flask). The complete medium CgIII was used as the medium for the pre-culture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Kanamycin (25 mg/l) was added to this. The pre-culture was incubated for 24 hours at 33°C at 240 rpm on a shaking machine. A main culture was seeded from this pre-culture such that the initial OD (660 nm) of the main culture was 0.1 OD. Medium MM was used for the main culture.

| Medium MM | |
|---|---|
| CSL (corn steep liquor) | 5 g/l |
| MOPS | 20 g/l |
| Glucose (autoclaved separately) | 50g/l |
| Salts: | |
| (NH₄)₂SO₄) | 25 g/l / |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| CaCO₃ | 2.5 g/l |

The CSL, MOPS and the salt solution are brought to pH 7 with aqueous ammonia and autoclaved. The sterile substrate and vitamin solutions are then added, as well as the CaCO₃ autoclaved in the dry state. For culturing of DSM 5715, 0.1 g/l leucine was additionally added to the medium. For culturing of FERM BP-1763, 0.1 g/l isoleucine and 0.1 g/l methionine were additionally added to the medium.

Culturing is carried out in a 10 ml volume in a 100 ml conical flask with baffles. Kanamycin (25 mg/l) was added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of L-lysine and of L-valine formed was determined with an amino acid analyzer from Eppendorf-BioTronik (Hamburg, Germany) by ion exchange chromatography and post-column derivation with ninhydrin detection.

The results of the experiment are shown in Tables 1 and 2.

**Table 1**

| Strain | OD(660) | Lysine HCl g/l |
|---|---|---|
| DSM5715 | 7.5 | 13.01 |
| DSM5715::pCR2.1lysR3int | 7.6 | 15.04 |

**Table 2**

| Strain | OD(660) | Valine g/l |
|---|---|---|
| FERM BP-1763 | 12.1 | 7.49 |
| FERM BP-1763::pCR2.1lysR3int | 12.5 | 8.67 |

### Brief Description of the Figures:

Figure 1: Map of the plasmid pCR2.1lysR3int.

The abbreviations and designations used have the following meaning.
- KmR:: Kanamycin resistance gene
- EcoRI:: cleavage site of the restriction enzyme EcoRI
- lysR3int:: Internal fragment of the lysR3 gene
- ColE1 ori:: Replication origin of the plasmid ColE1

## Claims

1. An isolated coryneform bacterium in which the expression of a polynucleotide encoding a polypeptide having an amino acid sequence which is at least 90 % identical to the amino acid sequence set out in SEQ ID NO:2 is eliminated, wherein the polypeptide has the function of a transcription regulator of the lysR family.

2. The bacterium of claim 1, wherein said bacterium is of the species Corynebacterium glutamicum.

3. *.* Coryneform bacterium according to claim 1 or 2, wherein the polynucleotide is chosen from the group:
a) nucleotide sequence as set out in SEQ ID NO:1; or
b) nucleotide sequences corresponding to sequence SEQ ID NO:1 within the degeneracy of the genetic code; or
c) nucleotide sequences with neutral sense mutations in SEQ ID NO:1.

4. The bacterium of claim 1, wherein said polypeptide has the amino acid sequence set out in SEQ ID NO:2.

5. A process for the preparation of L-lysine or L-valine comprising:
fermentation of said amino acids producing bacterium according to any of the claims 1 to 4 in a medium.

6. A process for the preparation of L-lysine or L-valine comprising fermentation of coryneform bacteria in which the intracellular activity of the polypeptide having the amino acid sequence as set out in SEQ ID NO:2 is eliminated, particularly the expression of polynucleotides chosen from the group:
a) nucleotide sequence as set out in SEQ ID NO: 1; or
b) nucleotide sequences corresponding to sequence SEQ ID NO:1 within the degeneracy of the genetic code; or
c) nucleotide sequences with neutral sense mutations in SEQ ID NO:1
encoding said polypeptide with the function of a transcription regulator of the lysR family, are eliminated.

7. A process according to claims 5 or 6,
a) fermentation of said coryneform bacteria which produce the desired L-amino acid,
b) concentration of said amino acid in the medium or in the cells of the bacteria and
c) isolation of the L-amino acid.

8. The process according to claims 5 to 7, wherein bacteria in which further genes of the biosynthesis pathway of the desired L-amino acid are additionally enhanced are employed.

9. The process according to claims 5 to 8, wherein bacteria in which the metabolic pathways which reduce the formation of the desired L-amino acid are eliminated are employed.

10. A process according to claim 7, wherein expression of the polynucleotide(s) which codes (code) for the lysR3 gene is eliminated.

11. A process according to claim 7, wherein the regulatory properties of the polypeptide for which the polynucleotide lysR3 codes are eliminated.

12. A process according to claim 8, wherein for the preparation of L-lysine, bacteria are fermented in which at the same time one or more of the genes chosen from the group consisting of
a) the dapA gene which codes for dihydrodipicolinate synthase,
b) the eno gene which codes for enolase,
c) the zwf gene which codes for the zwf gene product,
d) the pyc gene which codes for pyruvate carboxylase,
e) the lysE gene which codes for a protein for lysine export,
is or are enhanced, preferably over-expressed.

13. A process according to claim 9, wherein bacteria fermented, in which at the same time one or more of the genes chosen from the group consisting of:
a) the pck gene which codes for phosphoenol pyruvate carboxykinase,
b) the pgi gene which codes for glucose 6-phosphate isomerase,
c) the poxB gene which codes for pyruvate oxidase
is or are eliminated.

14. A process according to claim 8, wherein for the preparation of L-valine bacteria are fermented in which at the same time one or more of the genes chosen from the group consisting of
a) the ilvBN gene which codes for acetohydroxy-acid synthase,
b) the ilvD gene which codes for dihydroxy-acid dehydratase,
c) the mqo gene which codes for malate:quinone oxidoreductase
is or are overexpressed.

15. A process as claimed in one or more of the preceding claims, wherein microorganisms of the species Corynebacterium glutamicum are employed.

16. Vector for integration mutagenesis comprising the internal fragment of SEQ ID NO:1 set out in SEQ ID NO:3.

17. The vector pCR2.1lysR3int, deposited in E. coli strain TOP10F/pCR2.1lysR3int as DSM 13618.

18. Coryneform bacteria transformed with an integration vector which carries the polynucleotide set out in SEQ ID NO: 3.

## Patentansprüche

1. Isoliertes coryneformes Bakterium, bei dem die Expression eines Polynucleotids, das ein Polypeptid kodiert, das eine Aminosäuresequenz hat, die zu wenigstens 90 % identisch mit der Aminosäuresequenz in SEQ ID Nr. 2 ist, ausgeschaltet ist, wobei das Polypeptid die Funktion eines Transkriptionsregulators der lysR-Familie hat.

2. Bakterium nach Anspruch 1, wobei das genannte Bakterium zur Spezies Corynebacterium glutamicum gehört.

3. Coryneformes Bakterium nach Anspruch 1 oder 2, wobei das Polynucleotid ausgewählt ist aus der Gruppe bestehend aus:
a) der in SEQ ID Nr. 1 dargelegten Nucleotidsequenz oder
b) Nucleotidsequenzen, die der Sequenz SEQ ID Nr. 1 innerhalb der Degeneration des genetischen Codes entsprechen; oder
c) Nucleotidsequenzen mit neutralen Sinnmutationen in SEQ ID Nr. 1.

4. Bakterium nach Anspruch 1, wobei das genannte Polypeptid die in SEQ ID Nr. 2 dargelegte Aminosäuresequenz hat.

5. Verfahren zur Herstellung von L-Lysin oder L-Valin, umfassend das Fermentieren des genannten aminosäureproduzierenden Bakteriums nach einem der Ansprüche 1 bis 4 in einem Medium.

6. Verfahren zur Herstellung von L-Lysin oder L-Valin, umfassend das Fermentieren coryneformer Bakterien, in denen die intrazelluläre Aktivität des Polypeptids mit der in SEQ ID Nr. 2 dargelegten Aminosäuresequenz ausgeschaltet ist, insbesondere die Expression der Polynucleotide, ausgewählt aus der Gruppe bestehend aus:
a) der in SEQ ID Nr. 1 dargelegten Nucleotidsequenz oder
b) Nucleotidsequenzen, die der Sequenz SEQ ID Nr. 1 innerhalb der Degeneration des genetischen Codes entsprechen; oder
c) Nucleotidsequenzen mit neutralen Sinnmutationen in SEQ ID Nr. 1,
die das genannte Polypeptid mit der Funktion eines Transkriptionsregulators der lysR-Familie kodieren, ausgeschaltet ist.

7. Verfahren nach Anspruch 5 oder 6,
a) Fermentation der genannten coryneformen Bakterien, die die gewünschte L-Aminosäure produzieren,
b) Konzentration der genannten Aminosäure in dem Medium oder in den Zellen der Bakterien und
c) Isolierung der L-Aminosäure.

8. Verfahren nach den Ansprüchen 5 bis 7, wobei Bakterien verwendet werden, in denen weitere Gene des Biosynthesewegs der gewünschten L-Aminosäure zusätzlich verstärkt sind.

9. Verfahren nach den Ansprüchen 5 bis 8, wobei Bakterien verwendet werden, in denen die Stoffwechselwege, die die Bildung der gewünschten L-Aminosäure verringern, ausgeschaltet sind.

10. Verfahren nach Anspruch 7, wobei die Expression des/der Polynucleotids/Polynucleotide, das/die das lysR3-Gen kodiert/kodieren, ausgeschaltet ist.

11. Verfahren nach Anspruch 7, wobei die regulatorischen Eigenschaften des Polypeptids, das das Polynukleotid lysR3 kodiert, ausgeschaltet sind.

12. Verfahren nach Anspruch 8, wobei für die Herstellung von L-Lysin Bakterien fermentiert werden, in denen gleichzeitig eines oder mehrere der Gene, ausgewählt aus der Gruppe bestehend aus
a) dem die Dihydrodipicolinat-Synthase kodierenden Gen dapA,
b) dem die Enolase kodierenden Gen eno,
c) dem das zwf-Genprodukt kodierenden Gen zwf,
d) dem die Pyruvat-Carboxylase kodierenden Gen pyc,
e) dem ein Protein für den Lysin-Export kodierenden Gen lysE,
verstärkt, bevorzugt überexprimiert wird/werden.

13. Verfahren nach Anspruch 9, wobei Bakterien fermentiert werden, in denen gleichzeitig eines oder mehrere der Gene, ausgewählt aus der Gruppe bestehend aus:
a) dem die Phosphoenolpyruvat-Carboxykinase kodierenden Gen pck,
b) dem die Glucose-6-Phosphatisomerase kodierenden Gen pgi,
c) dem die Pyruvatoxidase kodierenden Gen poxB, ausgeschaltet wird/werden.

14. Verfahren nach Anspruch 8, wobei zur Herstellung von L-Valin Bakterien fermentiert werden, in denen gleichzeitig ein oder mehrere der Gene, ausgewählt aus der Gruppe bestehend aus:
a) dem die Acetohydroxysäuresynthase kodierenden ilvBN-Gen,
b) dem die Dihydroxysäuredehydratase kodierenden ilvD-Gen,
c) dem die Malat:Chinon-Oxidoreduktase kodierenden mqo-Gen
überexprimiert wird/werden.

15. Verfahren nach einem oder mehreren der vorherigen Ansprüche, wobei Mikroorganismen der Spezies Corynebacterium glutamicum verwendet werden.

16. Vektor zur Integrationsmutagenese, umfassend das interne Fragment von SEQ ID Nr.1, dargelegt in SEQ ID Nr. 3.

17. Vektor pCR2.1lysR3int, hinterlegt im E. coli Stamm TOP10F/pCR2.1lysR3int als DSM 13618.

18. Coryneforme Bakterien, transformiert mit einem Integrationsvektor, der das in SEQ ID Nr. 3 dargelegte Polynucleotid trägt.

## Revendications

1. Bactérie coryneforme isolée, dans laquelle l'expression d'un polynucléotide codant un polypeptide ayant une séquence d'acides aminés qui est au moins identique à 90 % à la séquence d'acides aminés figurant dans SEQ ID NO : 2 est éliminée,
**caractérisée en ce que**
le polypeptide a la fonction d'un régulateur de transcription de la famille lysR.

2. Bactérie de la revendication 1,
**caractérisée en ce que**
la bactérie est de l'espèce Corynebactérium glutamicum.

3. Bactérie coryneforme selon la revendication 1 ou 2,
**caractérisée en ce que**
le polynucléotide est choisi dans le groupe constitué par :
a) une séquence de nucléotide comme celle figurant dans SEQ ID NO:1 ;ou
b) des séquences de nucléotides correspondant à la séquence SEQ ID NO : 1 ; à l'intérieur de la dégénérescence du code génétique ; ou
c) des séquences de nucléotides avec des mutations signifiantes neutres dans SEQ ID NO : 1.

4. Bactérie de la revendication 1,
**caractérisée en ce que**
le polypeptide a la séquence d'acides aminés figurant dans SEQ ID NO : 2.

5. Procédé pour la préparation de L-lysine ou de L-valine comprenant :
la fermentation des acides aminés produisant la bactérie, dans un milieu, selon l'une quelconque des revendications 1 à 4.

6. Procédé pour la préparation de L-lysine ou de L-valine comprenant
la fermentation de bactéries coryneforme dans lesquelles l'activité intracellulaire du polypeptide ayant la séquence d'acides aminés figurant dans SEQ ID No : 2 est éliminée, en particulier on élimine l'expression des polynucléotides choisis dans le groupe comprenant :
a) une séquence de nucléotide comme celle figurant dans SEQ ID NO : 1 ; ou
b) des séquences de nucléotides correspondant à la séquence SEQ ID NO : 1 ; à l'intérieur de la dégénérescence du code génétique ; ou
c) des séquences de nucléotides avec des mutations signifiantes neutres dans SEQ ID NO : 1,
codant le polypeptide avec la fonction d'un régulateur de transcription de la famille lysR.

7. Procédé selon les revendications 5 à 6,
**caractérisé par** les étapes :
a) fermentation de la bactérie coryneforme qui produit l'acide aminé L désiré,
b) concentration de l'acide aminé dans le milieu ou dans les cellules de la bactérie et
c) isolement de l'acide aminé L.

8. Procédé selon les revendications 5 à 7,
caractérisé en ce que'
on utilise les bactéries dans lesquelles en outre d'autres gènes de la voie de biosynthèse de l'acide aminé L désiré sont en plus améliorées.

9. Procédé selon les revendications 5 à 8,
**caractérisé en ce qu'**
on utilise des bactéries dans lesquelles les voies de biosynthèse qui réduisent la formation de l'acide aminé L désiré sont éliminées.

10. Procédé selon la revendication 7,
**caractérisé en ce que**
l'expression du ou des polynucléotides qui code le gène lysR3 est éliminée.

11. Procédé selon la revendication 7,
**caractérisé en ce que**
les propriétés de régulateur du polypeptide que le polynucléotide code sont éliminées.

12. Procédé selon la revendication 8,
**caractérisé en ce que**
pour la préparation de L-lysine, les bactéries sont mises à fermenter en même temps qu'un ou plusieurs des gènes choisis dans le groupe comprenant :
a) le gène dapA qui code la synthase dihydropicolinate,
b) le gène eno qui code l'énolase,
c) le gène zwf qui code le produit du gène zwf,
d) le gène pyc qui code la carboxylase pyruvate,
e) le gène lyse qui code une protéine pour l'exportation de lysine,
est ou sont mis en valeur, de préférence, sur-exprimés.

13. Procédé selon la revendication 9,
**caractérisé en ce que**
les bactéries sont mises à fermenter en même temps qu'on élimine ou plusieurs des gènes choisis dans le groupe comprenant :
a) le gène pck qui code la carboxykinase pyruvate du phosphoénol,
b) le gène pgi qui code l'isomérase 6-phosphate du glucose
c) le gène poxB qui code l'oxydase pyruvate.

14. Procédé selon la revendication 8,
**caractérisé en ce que**
pour la préparation de L-lysine, les bactéries sont mises à fermenter en même temps qu'un ou plusieurs des gènes choisis dans le groupe comprenant :
a) le gène ilvBN qui code la synthase de l'acide acétohydroxy,
b) le gène ilvD qui code la déshydratase de l'acide acétohydroxy
c) le gène mqo qui code le malate : quinone oxydoréductase,
est ou sont sur-exprimés.

15. Procédé revendiqué dans l'une ou plusieurs des revendications précédentes,
**caractérisé en ce qu'**
on utilise des micro-organismes de l'espèce Corynebactérium glutamicum.

16. Vecteur pour la mutagénèse d'intégration comprenant le fragment interne de SEQ ID NO : 1. figurant dans SEQ ID NO : 3.

17. Vecteur pCR2.1lysR3int, déposé dans la souche E.coli TOP10F/pCR2.1lysR3int comme DSM 13618.

18. Bactéries coryneforme transformées par un vecteur d'intégration qui porte le polynucléotide figurant dans SEQ ID NO:3.


### SEQUENCE PROTOCOL

<110> Degussa AG
<120> New nucleotide sequences which code for the lysR3 gene
<130> 000183 BT
<140>
   <141>
<160> 3
<170> PatentIn Ver. 2.1
<210> 1
   <211> 1032
   <212> DNA
   <213> Corynebacterium glutamicum
<220>
   <221> CDS
   <222> (201)..(830)
   <223> lysR3 gene
<400> 1
   gatgatcaaa tacttctaga tgtcatcgat aatgggcagg gatttgatgt ggcagaagtg 60
   atccgtaaaa aatccattgg actgcccaca gcgcaacgcc gggctgaagg gctgggcgga 120
   acaataatta ttgaatctac aatcggatcg ggaactggaa tttccgcccg ttttccctat 180
   ccacaaaagg accaagataa gtg atc cgt att ctg ttg gct gat gat cat ccc 233
                         Met Ile Arg Ile Leu Leu Ala Asp Asp His Pro
                          1               5                  10
   gtt gtt cgc gca ggc ctt gcc tcc ttg ctg gtg agt gaa gat gat ttt   281
   Val Val Arg Ala Gly Leu Ala Ser Leu Leu Val Ser Glu Asp Asp Phe
                15                  20                  25
   gag ata gtg gac atg gtg ggc acc cca gat gat gcc gtt gcg cgc gcc   329
   Glu Ile Val Asp Met Val Gly Thr Pro Asp Asp Ala Val Ala Arg Ala
            30                  35                  40
   gcg gaa ggc ggg gtg gat gtg gtg ttg atg gat ctg cgt ttt ggt gat   377
   Ala Glu Gly Gly Val Asp Val Val Leu Met Asp Leu Arg Phe Gly Asp
        45                  50                  55
   caa cca ggc atc gag gtc gcc ggc ggg gta gag gca acg cgt cgc atc   425
   Gln Pro Gly Ile Glu Val Ala Gly Gly Val Glu Ala Thr Arg Arg Ile
    60                  65                  70                  75
   cgt gcg ctg gac aac ccg cca cag gta ctg gtg gtg acc aac tac tcc   473
   Arg Ala Leu Asp Asn Pro Pro Gln Val Leu Val Val Thr Asn Tyr Ser
                    80                  85                  90
   aca gac ggc gat gtg gtg ggc gca gta tct gct ggt gcc gtg ggg tat   521
   Thr Asp Gly Asp Val Val Gly Ala Val Ser Ala Gly Ala Val Gly Tyr
                95                 100                 105
   ttg ctc aaa gat agc tcc cca gaa gat ctc att gcc ggt gtt cgc gat    569
   Leu Leu Lys Asp Ser Ser Pro Glu Asp Leu Ile Ala Gly Val Arg Asp
           110                 115                 120
   gcc gcg cgg gga gaa tca gtg ctt tca aag cag gtc gcc agc aag atc    617
   Ala Ala Arg Gly Glu Ser Val Leu Ser Lys Gln Val Ala Ser Lys Ile
       125                 130                 135
   atg ggg cgg atg aac aac ccc atg act gct ctc agt gcc aga gaa att    665
   Met Gly Arg Met Asn Asn Pro Met Thr Ala Leu Ser Ala Arg Glu Ile
   140                 145                 150                 155
   gaa gtg ctg tcc ttg gtg gcg caa ggg caa agc aat aga gaa atc ggc    713
   Glu Val Leu Ser Leu Val Ala Gln Gly Gln Ser Asn Arg Glu Ile Gly
                   160                 165                 170
   aag aaa ctt ttc ctc act gag gcc acg gtg aaa agt cac atg ggg cat    761
   Lys Lys Leu Phe Leu Thr Glu Ala Thr Val Lys Ser His Met Gly His
               175                 180                 185
   gtg ttc aac aag ctg gat gtc acc tct aga aca gct gcg gta gct gaa    809
   Val Phe Asn Lys Leu Asp Val Thr Ser Arg Thr Ala Ala Val Ala Glu
           190                 195                 200
   gcc aga cag cgc gga att atc tagacgcaca cgtgttggta accgatcaca       860
   Ala Arg Gln Arg Gly Ile Ile
       205                 210
   ccagcgcacg ctgctaatct tcactccatg aacaaggtgc agcgcaggtc actgatggcg 920
   ttgtgcatga cggtggcatt tgctggagga agcctgaccg cgtgcacacc tcgtcctgat 980
   accgcagacc ccatcgcaga ggaattcctt caagcttggg catcgcaaga tt          1032
<210> 2
   <211> 210
   <212> PRT
   <213> Corynebacterium glutamicum
<400> 2
   Met Ile Arg Ile Leu Leu Ala Asp Asp His Pro Val Val Arg Ala Gly
   1               5                   10                  15
   Leu Ala Ser Leu Leu Val Ser Glu Asp Asp Phe Glu Ile Val Asp Met
               20                  25                  30
   Val Gly Thr Pro Asp Asp Ala Val Ala Arg Ala Ala Glu Gly Gly Val
           35                  40                  45
   Asp Val Val Leu Met Asp Leu Arg Phe Gly Asp Gln Pro Gly Ile Glu
       50                  55                  60
   Val Ala Gly Gly Val Glu Ala Thr Arg Arg Ile Arg Ala Leu Asp Asn
   65                  70                  75                  80
   Pro Pro Gln Val Leu Val Val Thr Asn Tyr Ser Thr Asp Gly Asp Val
                   85                  90                  95
   Val Gly Ala Val Ser Ala Gly Ala Val Gly Tyr Leu Leu Lys Asp Ser
               100                 105                 110
   Ser Pro Glu Asp Leu Ile Ala Gly Val Arg Asp Ala Ala Arg Gly Glu
           115                 120                 125
   Ser Val Leu Ser Lys Gln Val Ala Ser Lys Ile Met Gly Arg Met Asn
       130                 135                 140
   Asn Pro Met Thr Ala Leu Ser Ala Arg Glu Ile Glu Val Leu Ser Leu
   145                 150                 155                 160
   Val Ala Gln Gly Gln Ser Asn Arg Glu Ile Gly Lys Lys Leu Phe Leu
                   165                 170                 175
   Thr Glu Ala Thr Val Lys Ser His Met Gly His Val Phe Asn Lys Leu
               180                 185                 190
   Asp Val Thr Ser Arg Thr Ala Ala Val Ala Glu Ala Arg Gln Arg Gly
           195                 200                 205
   Ile Ile
       210
<210> 3
   <211> 323
   <212> DNA
   <213> Corynebacterium glutamicum
<220>
   <223> lysR3int
<400> 3
   gatgtggtgt tgatggatct gcgttttggt gatcaaccag gcatcgaggt cgccggcggg 60
   gtagaggcaa cgcgtcgcat ccgtgcgctg gacaacccgc cacaggtact ggtggtgacc 120
   aactactcca cagacggcga tgtggtgggc gcagtatctg ctggtgccgt ggggtatttg 180
   ctcaaagata gctccccaga agatctcatt gccggtgttc gcgatgccgc gcggggagaa 240
   tcagtgcttt caaagcaggt cgccagcaag atcatggggc ggatgaacaa ccccatgact 300
   gctctcagtg ccagagaaat tga                                          323